(19) Europäisches Patentamt
European Patent Office
Office européen des brevets

(11) Veröffentlichungsnummer: **0 031 885**
**B1**

(12) # EUROPÄISCHE PATENTSCHRIFT

(45) Veröffentlichungstag der Patentschrift: **27.06.84**

(21) Anmeldenummer: **80107005.3**

(22) Anmeldetag: **13.11.80**

(51) Int. Cl.³: **C 07 D 311/30,** C 07 D 311/22, C 07 D 311/32, C 07 D 311/36, C 07 D 311/58, C 07 D 311/60, C 07 D 211/22, C 07 D 405/12, C 07 D 407/04, C 07 C 93/02, A 61 K 31/35

(54) **Basische Äther, diese enthaltende pharmazeutische Zubereitungen und Verfahren zu ihrer Herstellung.**

(30) Priorität: **13.12.79 DE 2950135**

(43) Veröffentlichungstag der Anmeldung: **15.07.81 Patentblatt 81/28**

(45) Bekanntmachung des Hinweises auf die Patenterteilung: **27.06.84 Patentblatt 84/26**

(84) Benannte Vertragsstaaten: **AT BE CH DE FR GB IT LI NL SE**

(56) Entgegenhaltungen:
**BE - A - 666 541**
**DE - C - 1 054 091**
**GB - A - 1 154 119**

**JOURNAL FÜR PRAKTISCHE CHEMIE, Band 311, 1969 Leipzig J. KLOSA *"Synthesen neuer Aminoalkyläther von 7-Hydroxy-chromonen"* Seiten 183 bis 186**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung, Frankfurter Strasse 250, D-6100 Darmstadt (DE)**

(72) Erfinder: **Hausberg, Hans-Heinrich, Dr., Odenwaldstrasse 30, 6105 Ober-Ramstadt (DE)**
Erfinder: **Prücher, Helmut, Bahnhofstrasse 22, D-6148 Heppenheim (DE)**
Erfinder: **Uhl, Jürgen, Dr., Odenwaldstrasse 49, D-6104 Seeheim 1 (DE)**
Erfinder: **Seyfried, Christoph, Dr., Am Landbach 9, D-6104 Seeheim-Jugenheim (DE)**
Erfinder: **Minck, Klaus, Dr., Büchestrasse 8, D-6105 Ober-Ramstadt. (DE)**

**EP 0 031 885 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue basische Äther der allgemeinen Formel I

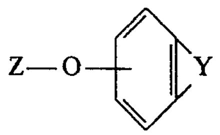

(I)

worin

| | |
|---|---|
| Z | $CH_3-NR^2-CH_2CH_2-CHR^1-$, (1-$R^2$-3-piperidyl)$-CHR^3-$, (1-$R^2$-2-piperidyl)$-CH_2-CHR^3-$ oder 1-$R^2$-3-$R^4$-4-hexahydroazepinyl, |
| $R^1$ | Cyclopropyl oder Ar, |
| $R^2$ | H, Alkyl mit 1–4 C-Atomen, Allyl, Cycloalkylalkyl mit 4–8 C-Atomen oder Benzyl, |
| $R^3$ | H oder Ar, |
| $R^4$ | H oder Alkyl mit 1–4 C-Atomen, |
| Y | $-O-CHQ^1-CHQ^2-CH_2-$, $-O-CHQ^1-CHQ^2-CO-$, $-O-CQ^1{=}CQ^2-CO-$ oder $-CH_2-CHQ^1-CHQ^2-CO-$, |
| $Q^1$ und $Q^2$ | jeweils H, Alkyl mit 1–4 C-Atomen, Cycloalkyl mit 3–6 C-Atomen oder Ar und |
| Ar | Phenyl oder durch F, Cl, Alkoxy oder Alkylthio mit jeweils 1–4 C-Atomen, Methylendioxy oder $CF_3$ substituiertes Phenyl |

bedeuten, worin jedoch

$R^1$ Cyclopropyl oder durch F, Cl, Alkoxy oder Alkylthio mit jeweils 1–4 C-Atomen, Methylendioxy oder $CF_3$ substituiertes Phenyl

bedeutet, wenn gleichzeitig

Z eine $(CH_3)_2N-CH_2CH_2-CHR^1$-Gruppe in 7-Stellung und
Y $-O-C(C_6H_5){=}CH-CO-$ oder $-O-C(C_6H_5){=}C(CH_3)-CO-$

bedeuten, sowie ihre physiologisch unbedenklichen Säureadditionssalze.

Der Erfindung lag die Aufgabe zugrunde, neue Verbindungen aufzufinden, die zur Herstellung von Arzneimitteln verwendet werden können. Diese Aufgabe wurde durch die Bereitstellung der Verbindungen der Formel I gelöst.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem antidepressive Wirkungen. Im einzelnen kann eine reserpinantagonistische Wirkung (feststellbar z. B. an Mäusen gegenüber Reserpin in Anlehnung an die Methode von Askew, Life Science, Bd. 10 [1963], Seiten 725–730), eine antikataleptische Wirkung (feststellbar z. B. an Ratten gegenüber Tetrabenazin in Anlehnung an die Methode von Giurgea et al., Medicina Experimentalis, Bd. 9, [1963], Seiten 249–262) und eine antiptotische Wirkung (feststellbar z. B. gegenüber Reserpin in Anlehnung an die Methodik von Domenjoz und Theobald, Arch. int. pharmacodyn., Bd. 120 [1959], Seite 450 ff., mit der Bewertung nach Rubin et al., J. Pharmacol. Exp. Therap., Bd. 120 [1957], Seiten 125–136) nachgewiesen werden. Weiterhin kann die Wirkung des 5-Hydroxytryptophans bei Mäusen (Methode: ähnlich wie Ross et al., Acta pharmacol. et toxicol., Bd. 39 [1976], Seiten 152–166) potenziert werden und die zentralen Auswirkungen einer Erregung und Temperatursteigerung, die D-Amphetaminsulfat (z. B. 1,5 mg/kg s.c., 1 Stunde nach der Prüfsubstanz verabfolgt, die ebenfalls s.c. appliziert wird) und Aggregation (Zusammensetzung von 5 Ratten in einem Glas) auslösen (Methodik nach Müller-Calgan et al. in Zippel, H. P. (Ed.): Memory and Transfer of Information, Plenum Press, New York–London, 1973, Seiten 87–125) können, erhöht und/oder verlängert werden. Die Substanzen haben einen Einfluß auf die biogenen Amine des ZNS. So führen sie z. B. in vitro zur Aufnahmehemmung von Noradrenalin, 5-Hydroxytryptamin und Dopamin (Methodik: Kannengießer et al., Biochem. Pharmacol., Bd. 22 (1973), Seiten 73–84) in Synaptosomen und hemmen in vivo (Methodik: Carlsson et al., Europ. J. Pharmacol., Bd. 5 [1969], Seiten 357–366; 367–373) die durch Tyraminderivate induzierte Katecholamin- und Serotonin-Freisetzung im Gehirn. Weiterhin treten blutdrucksenkende und spasmolytische Wirkungen auf, die nach hierfür geläufigen Methoden ermittelt werden können.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Aus Journal für praktische Chemie, Band 311, Seiten 183 bis 186 (1969) sind ähnliche Verbindungen bekannt, insbesondere das 7-(1-Phenyl-3-dimethylamino-propoxy)-flavon und das 3-Methyl-7-(1-phenyl-3-dimethylamino-propoxy)-flavon. Diese Substanzen wurden auf Analgesie, Spasmolyse, Coronaraktivität und Permeabilitätswirkung getestet und sie zeigten dabei keine pharmakodynamisch brauchbaren Eigenschaften. Im Hinblick auf diese Feststellung waren die wertvollen pharmakologischen Eigenschaften der vorliegenden neuen Verbindungen daher besonders überraschend.

Gegenstand der Erfindung sind die basischen Äther der Formel I sowie ihre physiologisch unbedenklichen Säureadditionssalze.

In den Resten $R^2$, $R^4$, $Q^1$ und $Q^2$ bedeutet Alkyl vorzugsweise Methyl, ferner auch Äthyl, n-Propyl, Isopropyl, n-Butyl, Isobutyl, sek.-Butyl oder tert.-Butyl. Cycloalkyl (in den Resten $Q^1$ und $Q^2$) bedeutet vorzugsweise Cyclopropyl, Cyclobutyl, Cyclopentyl oder Cyclohexyl, ferner auch 1- oder 2-Methylcyclopropyl, 1-, 2- oder 3-Methylcyclobutyl, 1-, 2- oder 3-Methylcyclopentyl, 1- oder 2-Äthylcyclopropyl usw. Alkoxy (im Rest Ar) ist vorzugsweise Methoxy, ferner auch Äthoxy, n-Propoxy, Isopropoxy, n-Butoxy, Isobutoxy, sek.-Butoxy oder tert.-Butoxy. Cycloalkylalkyl (im Rest $R^2$) ist vorzugsweise Cyclopropylmethyl, aber auch z. B. 1- oder 2-Cyclopropyläthyl, 1-, 2- oder 3-Cyclopropylpropyl, 1-Cyclopropyl-1-methyl-äthyl, 1-, 2-, 3- oder 4-Cyclopropylbutyl, 1-Cyclopropyl-1-methylpropyl, 1-, 2-, 3-, 4- oder 5-Cyclopropylpentyl, Cyclobutylmethyl, 1- oder 2-Cyclobutyläthyl, 1-, 2- oder 3-Cyclobutylpropyl, 1-, 2-, 3- oder 4-Cyclobutylbutyl, Cyclopentylmethyl, 1- oder 2-Cyclopentyläthyl, 1-, 2- oder 3-Cyclopentylpropyl, Cyclohexylmethyl, 1- oder 2-Cyclohexyläthyl oder Cycloheptylmethyl. Alkylthio (im Rest Ar) ist vorzugsweise Methylthio, aber auch Äthylthio, n-Propylthio, Isopropylthio, n-Butylthio, Isobutylthio, sek.-Butylthio oder tert.-Butylthio.

Der Rest Ar ist vorzugsweise Phenyl, in zweiter Linie p-Fluorphenyl, p-Chlorphenyl, p-Methoxyphenyl, 3,4-Methylendioxyphenyl oder m-Trifluormethylphenyl, weiterhin o- oder m-Fluorphenyl, o- oder m-Chlorphenyl, o- oder m-Methoxyphenyl, o-, m- oder p-Methylthiophenyl, 2,3-Methylendioxyphenyl, o- oder p-Trifluormethylphenyl. Falls mehrere Reste Ar im gleichen Molekül vorhanden sind, so können sie gleich oder voneinander verschieden sein.

Im einzelnen steht $R^1$ vorzugsweise für Phenyl, p-Fluorphenyl, p-Chlorphenyl oder p-Methoxyphenyl; $R^2$ vorzugsweise für H oder Methyl, in zweiter Linie vorzugsweise für Äthyl, Allyl, Cyclopropylmethyl oder Benzyl; $R^3$ vorzugsweise für H oder Phenyl; $R^4$ vorzugsweise für H oder Methyl.

Dementsprechend bedeutet der Rest Z vorzugsweise 1-Phenyl-3-dimethylaminopropyl, in zweiter Linie vorzugsweise

1-Phenyl-3-methylaminopropyl, 1-Phenyl-3-N-benzyl-N-methylamino-propyl,
(3-Piperidyl)-methyl, $\alpha$-(3-Piperidyl)-benzyl,
(1-Methyl-3-piperidyl)-methyl, $\alpha$-(1-Methyl-3-piperidyl)-benzyl,
2-(2-Piperidyl)-äthyl, 1-Phenyl-2-(2-piperidyl)-äthyl,
2-(1-Methyl-2-piperidyl)-äthyl, 1-Phenyl-2-(1-methyl-2-piperidyl)-äthyl,
4-Hexahydro-azepinyl, 1-Methyl-4-hexahydroazepinyl,
3-Methyl-4-hexahydroazepinyl, 1,3-Dimethyl-4-hexahydroazepinyl,

ferner z. B.

1-Phenyl-3-methyläthyl-amino-propyl, 1-Phenyl-3-(N-methyl-N-allylamino)-propyl,
1-Phenyl-3-(N-methyl-N-cyclopropylmethylamino)-propyl,
1-Äthyl-3-piperidyl)-methyl, $\alpha$-(1-Äthyl-3-piperidyl)-benzyl,
(1-Allyl-3-piperidyl)-methyl, $\alpha$-(1-Allyl-3-piperidyl)-benzyl,
(1-Cyclopropylmethyl-3-piperidyl)-methyl, $\alpha$-(1-Cyclopropylmethyl-3-piperidyl)-benzyl,
(1-Benzyl-3-piperidyl)-methyl, $\alpha$-(1-Benzyl-3-piperidyl)-benzyl,
2-(1-Äthyl-2-piperidyl)-äthyl, 1-Phenyl-2-(1-äthyl-2-piperidyl)-äthyl,
2-(1-Allyl-2-piperidyl)-äthyl, 1-Phenyl-2-(1-allyl-2-piperidyl)-äthyl,
2-(1-Cyclopropylmethyl-2-piperidyl)-äthyl,
1-Phenyl-2-(1-cyclopropylmethyl-2-piperidyl)-äthyl,
2-(1-Benzyl-2-piperidyl)-äthyl, 1-Phenyl-2-(1-benzyl-2-piperidyl)-äthyl,
3-Äthyl-4-hexahydroazepinyl, 1-Methyl-3-äthyl-4-hexahydroazepinyl,
1-Äthyl-, 1-Allyl-, 1-Cyclopropylmethyl- oder 1-Benzyl-4-hexahydroazepinyl,
1-Cyclopropyl-3-methylamino-propyl, 1-p-Fluorphenyl-3-methylamino-propyl,
1-p-Chlorphenyl-3-methylamino-propyl, 1-m-Trifluormethyl-phenyl-3-methylamino-propyl,
1-Cyclopropyl-3-dimethylamino-propyl, 1-p-Fluorphenyl-3-dimethylamino-propyl,
1-p-Chlorphenyl-3-dimethylamino-propyl, 1-m-Trifluormethylphenyl-3-dimethylamino-propyl.

Die Reste $Q^1$ und $Q^2$ können gleich oder verschieden sein. Vorzugsweise steht der eine dieser Reste für H oder Alkyl mit 1—3 C-Atomen, der andere für H oder Ar, wobei Ar insbesondere Phenyl, p-Fluorphenyl, p-Chlorphenyl, p-Methoxyphenyl, 3,4-Methylendioxyphenyl oder m-Trifluormethylphenyl bedeutet.

Der Rest Y bedeutet dementsprechend vorzugsweise $-O-CHQ^3-CHQ^4-CH_2-$ (worin die Reste $Q^3$ und $Q^4$ jeweils H oder Alkyl mit 1—3 C-Atomen, der eine dieser Reste jedoch vorzugsweise H bedeutet;

Chromane), $-O-CHAr-CHQ^4-CH_2-$ (Flavane), $-O-CHQ^3-CHAr-CH_2-$ (Isoflavane), $-O-CHQ^3-CHQ^4-CO-$ (Chromanone), $-O-CHAr-CHQ^4-CO-$ (Flavanone), $-O-CHQ^3-CHAr-CO-$ (Isoflavanone), $-O-CQ^3{=}CQ^4-CO-$ (Chromone), $-O-CAr{=}CQ^4-CO-$ (Flavone), $-O-CQ^3{=}CAr-CO-$ (Isoflavone) oder $-CH_2-CHQ^3-CHQ^4-CO-$ (Tetralone).

Unter diesen sind die Isoflavone, in zweiter Linie die Tetralone und die Flavone besonders bevorzugt.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis Ic ausgedrückt werden, die der Formel I entsprechen und worin die nicht bezeichneten Reste die bei Formel I angegebene Bedeutung haben, worin jedoch in Ia

- Z $CH_3-NR^2-CH_2CH_2-CHR^1-$,
- $R^1$ Phenyl,
- $R^2$ H, Methyl oder Benzyl,
- Y $-O-CH_2CH_2CH_2-$, $-O-CHAr-CH_2CH_2-$, $-O-CH_2-CH(C_6H_5)-CH_2-$, $-O-CH_2CH_2-CO-$, $-O-CHAr-CHQ^4-CO-$, $-O-CAr{=}CQ^4-CO-$, $-O-CH{=}CAr-CO-$ oder $-CH_2CH_2CH_2-CO-$,
- Ar Phenyl, p-Fluorphenyl, p-Chlorphenyl, p-Methoxyphenyl, 3,4-Methylendioxyphenyl oder m-Trifluormethylphenyl und
- $Q^4$ H oder Alkyl mit 1–3 C-Atomen bedeuten;

in Ib

- Z $CH_3-NR^2-CH_2CH_2-CHR^1$,
- $R^1$ Phenyl,
- $R^2$ H, Methyl oder Benzyl und
- Y $-O-C(C_6H_5){=}CH-CO-$, $-O-CH{=}C(C_6H_5)-CO-$ oder $-CH_2CH_2CH_2-CO-$ bedeuten;

in Ic

- Z $CH_3-NR^2-CH_2CH_2-CHR^1-$,
- $R^1$ Phenyl,
- $R^2$ H, Methyl oder Benzyl und
- Y $-O-CH{=}C(C_6H_5)-CO-$ bedeuten.

Die Verbindungen der Formel I können ein oder mehrere asymmetrische Kohlenstoffatome besitzen. Sie können daher als Racemate, falls mehrere asymmetrische Kohlenstoffatome vorhanden sind, auch als Gemische mehrerer Racemate sowie in verschiedenen optisch-aktiven Formen vorliegen.

Gegenstand der Erfindung ist ferner ein Verfahren zur Herstellung der Verbindungen der Formel I sowie ihrer physiologisch unbedenklichen Säureadditionssalze, dadurch gekennzeichnet, daß man ein Phenol der allgemeinen Formel II

$$HO-G \qquad (II)$$

worin

G die Gruppe

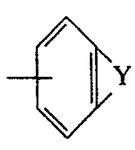

bedeutet, oder eines seiner Salze mit einem Amin der allgemeinen Formel III

$$Z-X \qquad (III)$$

worin

- X Cl, Br, J oder OH bedeutet und
- Z die angegebene Bedeutung hat

oder mit einem seiner reaktionsfähigen Derivate umsetzt und daß man gegebenenfalls in einer erhaltenen Verbindung der Formel I eine sekundäre Aminogruppe durch Behandeln mit einem alkylierenden, allylierenden, cycloalkylierenden oder benzylierenden Mittel in die entsprechende tertiäre Aminogruppe oder eine N-Benzylgruppe durch Behandeln mit einem Reduktionsmittel in eine NH-Gruppe und/oder eine erhaltene Base der Formel I durch Behandeln mit einer Säure in eines ihrer physiologisch unbedenklichen Säureadditionssalze umwandelt.

Die Herstellung der Verbindungen der Formel I erfolgt im übrigen nach an sich bekannten Methoden, wie sie in der Literatur (z. B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe der Formeln II und III können gewünschtenfalls auch in situ gebildet werden, derart, daß man sie aus dem Reaktionsgemisch nicht isoliert, sondern sofort weiter zu den Verbindungen der Formel I umsetzt.

Die Phenole der Formel II sind in der Regel bekannt. Falls sie neu sind, können sie nach an sich bekannten Methoden hergestellt werden, z. B. durch Spaltung entsprechender Benzyl- oder Methyläther.

In den Basen der Formel III bedeutet der Rest X vorzugsweise Cl oder Br. Reaktionsfähige Derivate der Basen der Formel III sind insbesondere die reaktionsfähigen Ester der Alkohole der Formel III (X = OH), vorzugsweise die entsprechenden Alkylsulfonate (worin die Alkylgruppe 1—6 C-Atome besitzt) und die entsprechenden Arylsulfonate (worin die Arylgruppe 6—10 C-Atome besitzt), z. B. die entsprechenden Methan-, Benzol-, p-Toluol- oder Naphthalin-1- oder -2-sulfonate.

Die Basen der Formel III sind teilweise bekannt. Die bisher noch nicht bekannten Basen der Formel III können nach an sich bekannten Methoden in Analogie zu bekannten Verbindungen hergestellt werden. So sind die Verbindungen der Formel III (X = OH) z. B. durch Reduktion entsprechender Ester oder Ketone vom Typ

$CH_3—NR^2—CH_2CH_2—COR^1$, (1-$R^2$-3-piperidyl)—COOAlkyl,
(1-$R^2$-3-piperidyl)—CO—$R^3$, (1-$R^2$-2-piperidyl)—$CH_2$—COOAlkyl,
(1-$R^2$-2-piperidyl)—$CH_2$—CO—$R^3$ oder 1-$R^2$-3-$R^4$-hexahydroazepin-4-on

erhältlich, die Verbindungen der Formel III (X = Cl, Br oder J) aus den Alkoholen mit anorganischen Halogeniden wie $SOCl^2$, $PBr^3$ oder HJ, die Sulfonate durch Veresterung der Alkohole mit den entsprechenden Sulfonylchloriden. Die tertiären unter den Aminen der Formel III (in denen $R^2$ nicht H bedeutet) sind auch aus den sekundären Aminen (III, $R^2$ = H) durch Alkylierung, Allylierung, Cycloalkylalkylierung oder Benzylierung zugänglich. Umgekehrt können die sekundären Amine (III, $R^2$ = H) aus den entsprechenden N-Alkylderivaten (III, $R^2$ = Alkyl mit 1—4 C-Atomen) durch Desalkylierung mit Chlorameisensäureäthylester erhalten werden. Weiterhin können die Amine der Formel III durch Reduktion entsprechender Pyridine oder Azepine hergestellt werden. So kann man z. B. 2-Methylpyridin mit $C_6H_5Li$ metallieren, anschließend mit Benzaldehyd zu 1-Phenyl-2-(2-pyridyl)-äthanol umsetzen und dieses zu 1-Phenyl-2-(2-piperidyl)-äthanol reduzieren. 1-Methyl-4-hydroxy-hexahydroazepin ist zugänglich durch Ringerweiterung von 1-Methyl-4-piperidon mit $CH_2N_2$ zu 1-Methyl-hexahydroazepin-4-on und Reduktion mit $NaBH_4$.

Vor der Umsetzung mit III wird das Phenol II zweckmäßig zunächst in ein Salz übergeführt, insbesondere in ein Metallsalz, z. B. ein Alkalimetallsalz (Li-, Na- oder K-Salz) oder Thalliumsalz. Man kann das Phenol mit einem metallsalz-bildenden Reagenz umsetzen, z. B. einem Alkalimetall (z. B. Na), einem Alkalimetallhydrid oder -amid (z. B. LiH, NaH, $NaNH_2$ oder $KNH_2$), einem Metallalkoholat (worin der Alkohol-Teil vorzugsweise 1—4 C-Atome besitzt, z. B. Lithium-, Natrium-, Kalium- oder Thalliummethylat, -äthylat oder -tert.-butylat), einer Organometallverbindung (z. B. Butyllithium, Phenyllithium oder Phenylnatrium), einem Metallhydroxid, -carbonat oder -bicarbonat (z. B. des Li, Na, K oder Ca). Die Herstellung des Phenolats wird vorteilhafterweise in Gegenwart eines Lösungsmittels oder Lösungsmittelgemischs vorgenommen. Geeignete Lösungsmittel sind z. B. Kohlenwasserstoffe (wie Hexan, Benzol, Toluol oder Xylol), halogenierte Kohlenwasserstoffe (wie $CH_2Cl_2$, $CHCl_3$ oder $CCl_4$), Äther (wie Diäthyläther, Diisopropyläther, Tetrahydrofuran (THF), Dioxan oder Diäthylenglykoldimethyläther), Amide wie Dimethylformamid (DMF), Alkohole (wie Methanol oder Aethanol), Ketone (wie Aceton oder Butanon).

Das Phenol II oder dessen Salz wird mit dem Amin III vorzugsweise in Gegenwart eines Verdünnungsmittels umgesetzt, z. B. desjenigen Lösungsmittels, das für die Herstellung des Salzes verwendet worden ist, das jedoch durch ein anderes Lösungsmittel ersetzt oder mit einem solchen verdünnt sein kann. Eine besondere Variante besteht darin, zweiphasig (z. B. in $CH_2Cl_2$/wässeriger Natronlauge) zu arbeiten, wobei auch ein Katalysator (z. B. ein Kronenäther, ein Ammoniumsalz wie ein Trialkylbenzylammoniumhalogenid oder ein Phosphoniumsalz) zugesetzt werden kann. Die Umsetzung wird in der Regel bei Temperaturen zwischen —20° und 180° durchgeführt, vorzugsweise zwischen 20 und 160°.

Das Phenolat kann auch in situ gebildet werden. In diesem Falle läßt man das Phenol II und das Amin III miteinander in Gegenwart einer Base reagieren.

Eine Variante der Reaktion besteht in der Umsetzung eines Phenols der Formel II mit einem Hydroxyamin der Formel III (X = OH) in Gegenwart eines Dehydratisierungsmittels, z. B. eines Azodicarbonsäuredialkylesters in Anwesenheit von Triphenylphosphin in einem inerten Lösungsmittel wie THF bei −10 bis +30°.

Weiterhin kann man gewünschtenfalls ein erhaltenes sekundäres Amin der Formel I ($R^2$ = H) am Stickstoffatom alkylieren, wobei man tertiäre Amine der Formel I ($R^2$ = Alkyl mit 1—4 C-Atomen) erhält. Als N-Alkylierungsmittel eignen sich z. B. die entsprechenden Alkylhalogenide, z. B. Methylchlorid, Methylbromid, Methyljodid, Äthylchlorid, Äthylbromid, Äthyljodid, n-Propylchlorid, -bromid oder -jodid usw., ferner die entsprechenden Dialkylsulfate wie Dimethylsulfat und die entsprechenden Sulfonsäurealkylester wie p-Toluolsulfonsäure-methylester. Eine Methylgruppe kann ferner beispielsweise durch Behandeln mit Ameisensäure und wässeriger Formaldehydlösung eingeführt werden, zweckmäßig durch mehrstündiges Erhitzen auf Temperaturen zwischen 50 und 100°. Allgemein wird die N-Alkylierung zweckmäßig in Gegenwart oder Abwesenheit eines inerten Lösungsmittels bei Temperaturen zwischen 0 und 120°, vorzugsweise zwischen 40 und 100° vorgenommen, wobei auch ein Katalysator zugegen sein kann, vorzugsweise eine Base wie Kalium-tert.-butylat.

Eine Alkylierung gelingt auch durch Behandeln einer sekundären Base I ($R^2$ = H) mit einem Aldehyd oder Keton in Gegenwart von Wasserstoff und einem Hydrierungskatalysator (z. B. Raney-Nickel) bei Temperaturen zwischen 50 und 100° und Drucken zwischen 1 und 200 at; so erhält man mit Aceton die entsprechende Isopropylverbindung I ($R^2$ = Isopropyl).

Eine Alkylierung ist auch mehrstufig möglich. Man kann beispielsweise eine Verbindung der Formel I ($R^2$ = H) zunächst in an sich bekannter Weise acylieren (z. B. durch Behandeln mit Acetanhydrid/Pyridin acetylieren) und das erhaltene N-Acylierungsprodukt (z. B. N-Acetylierungsprodukt) anschließend zum gewünschten tertiären Amin reduzieren, beispielsweise mit einem komplexen Metallhydrid wie $LiAlH_4$ in einem inerten Lösungsmittel wie Diäthyläther oder THF, vorzugsweise bei Temperaturen zwischen 20 und 60°.

In ganz analoger Weise kann ein sekundäres Amin der Formel I ($R^2$ = H) mit allylierenden, cycloalkylalkylierenden oder benzylierenden Mitteln (z. B. Allylchlorid oder -bromid, Cyclopropylmethylhalogeniden wie Cyclopropylmethylchlorid oder -bromid, Benzylhalogeniden wie Benzylchlorid oder -bromid) behandelt werden, wobei Verbindungen der Formel I ($R^2$ = Allyl, Cycloalkylalkyl mit 4—8 C-Atomen oder Benzyl) gebildet werden.

Weiterhin kann in einer Verbindung der Formel I ($R^2$ = Benzyl) die Benzylgruppe nach einer in der Literatur angegebenen Methode reduktiv entfernt werden, vorzugsweise durch Hydrogenolyse in Gegenwart eines Edelmetallkatalysators oder mit Hilfe eines desalkylierenden Agens, z. B. $ClCOC_2H_5$/NaOH.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z. B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diäthylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Äthansulfonsäure, Äthandisulfonsäure, 2-Hydroxyäthansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend eine Verbindung der Formel I und/oder eines ihrer physiologisch unbedenklichen Säureadditionssalze. Diese Zubereitungen können als Arzneimittel in der Human- oder Veterinärmedizin eingesetzt werden. Als Trägersubstanzen kommen organische oder anorganische Stoffe in Frage, die sich für die enterale (z. B. orale), parenterale oder topikale Applikation eignen und mit den neuen Verbindungen nicht reagieren, beispielsweise Wasser, pflanzliche Öle, Benzylalkohole, Polyäthylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topikale Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder

mehrere Vitamine.

Gegenstand der Erfindung sind ferner die Verbindungen der Formel I und ihrer physiologisch unbedenklichen Säureadditionssalze zur Verwendung bei der Bekämpfung von Krankheiten, insbesondere von Depressionen verschiedener Ätiologie und Symptomatologie, sowie bei der therapeutischen Behandlung des menschlichen oder tierischen Körpers.

Dabei werden die erfindungsgemäßen Substanzen in der Regel in Analogie zu bekannten, im Handel befindlichen Psychopharmaka (z. B. Imipramin) verabreicht, vorzugsweise in Dosierungen zwischen 2 und 500 mg, insbesondere zwischen 10 und 50 mg pro Dosierungseinheit. Die tägliche Dosierung liegt vorzugsweise zwischen 0,05 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Jede der in den folgenden Beispielen genannten Verbindungen der Formel I ist zur Herstellung von pharmazeutischen Zubereitungen besonders geeignet.

In den nachfolgenden Beispielen bedeutet ›übliche Aufarbeitung‹:

Man gibt, falls erforderlich, Wasser hinzu, extrahiert mit einem organischen Lösungsmittel wie Benzol, Chloroform oder Dichlormethan, trennt ab, trocknet die organische Phase über Natriumsulfat, filtriert, dampft ein und reinigt durch Chromatographie und/oder Kristallisation.

Die Rf-Werte wurden an Kieselgel mit Toluol : Triäthylamin (8 : 2) erhalten, sofern nicht anders angegeben.

## Beispiel 1

Eine Lösung von 23,8 g 7-Hydroxy-isoflavon in 250 ml 0,5 n äthanolischer KOH wird eingedampft, der Rückstand in 200 ml DMF gelöst, auf 150° erhitzt und unter Rühren mit einer Lösung von 20 g 1-Chlor-1-phenyl-3-dimethylamino-propan in 50 ml DMF versetzt. Man rührt 1,5 Stunden bei 150° und fällt durch Zugabe von Wasser 7-(1-Phenyl-3-dimethylamino-propoxy)-isoflavon. F. 128–130° (aus Aceton). Hydrochlorid, F. 259–260°.

## Beispiele 2 bis 220

Analog Beispiel 1 erhält man durch Verätherung der Na-Salze von

6- oder 7-Hydroxy-chroman, 6- oder 7-Hydroxyflavan, 6- oder 7-Hydroxyisoflavan,
6- oder 7-Hydroxychromanon, 6- oder 7-Hydroxyflavanon, 6- oder 7-Hydroxyisoflavanon,
6- oder 7-Hydroxychromon, 5-, 6-, 7- oder 8-Hydroxyflavon, 5-, 6-, 7- oder 8-Hydroxyisoflavon,
5-, 6- oder 7-Hydroxytetralon mit 1-Chlor-1-phenyl-3-methylaminopropan,
1-Chlor-1-phenyl-3-dimethylaminopropan, 1-Chlor-1-phenyl-3-N-benzyl-N-methylaminopropan,
3-Chlormethyl-piperidin, 3-(α-Chlorbenzyl)-piperidin, 1-Methyl-3-chlormethyl-piperidin,
1-Methyl-3-(α-chlorbenzyl)-piperidin, 2-(2-Chloräthyl)-piperidin,
2-(2-Chlor-2-phenyläthyl)-piperidin, 1-Methyl-2-(2-chloräthyl)-piperidin,
1-Methyl-2-(2-chlor-2-phenyläthyl)-piperidin, 1-Benzyl-2-(2-chloräthyl)-piperidin,
4-Chlorhexahydroazepin, 1-Methyl-4-chlor-hexahydroazepin,
3-Methyl-4-chlor-hexahydroazepin, 1,3-Dimethyl-4-chlorhexahydroazepin

oder den entsprechenden Bromverbindungen:

2. 6-(1-Phenyl-3-methylamino-propoxy)-chroman, Hydrochlorid, F. 164°.
3. 7-(1-Phenyl-3-methylamino-propoxy)-chroman.
4. 6-(1-Phenyl-3-dimethylamino-propoxy)-chroman, Hydrochlorid, F. 137–139°.
5. 7-(1-Phenyl-3-dimethylamino-propoxy)-chroman, Hydrochlorid, F. 182–183°.
6. 6-(1-Phenyl-3-N-benzyl-N-methylamino-propoxy)-chroman, Hydrochlorid, F. 139–140°.
7. 7-(1-Phenyl-3-N-benzyl-N-methylamino-propoxy)-chroman.
8. 6-[α-(1-Methyl-3-piperidyl)-benzyloxy]-chroman.
9. 7-[α-(1-Methyl-3-piperidyl)-benzyloxy]-chroman.
10. 6-[2-(1-Methyl-2-piperidyl)-äthoxy]-chroman, Hydrochlorid, F. 100–103°.
11. 7-[2-(1-Methyl-2-piperidyl)-äthoxy]-chroman.
12. 6-(1,3-Dimethyl-4-hexahydroazepinyloxy)-chroman.
13. 7-(1,3-Dimethyl-4-hexahydroazepinyloxy)-chroman.
14. 6-(1-Phenyl-3-methylamino-propoxy)-flavan.
15. 7-(1-Phenyl-3-methylamino-propoxy)-flavan.

16. 6-(1-Phenyl-3-dimethylamino-propoxy)-flavan.
17. 7-(1-Phenyl-3-dimethylamino-propoxy)-flavan.
18. 6-(1-Phenyl-3-N-benzyl-N-methylamino-propoxy)-flavan.
19. 7-(1-Phenyl-3-N-benzyl-N-methylamino-propoxy)-flavan.
20. 6-[α-(1-Methyl-3-piperidyl)-benzyloxy]-flavan.
21. 7-[α-(1-Methyl-3-piperidyl)-benzyloxy]-flavan.
22. 6-[2-(1-Methyl-2-piperidyl)-äthoxy]-flavan.
23. 7-[2-(1-Methyl-2-piperidyl)-äthoxy]-flavan.
24. 6-(1,3-Dimethyl-4-hexahydroazepinyloxy)-flavan.
25. 7-(1,3-Dimethyl-4-hexahydroazepinyloxy)-flavan.
26. 6-(1-Phenyl-3-methylamino-propoxy)-isoflavan.
27. 7-(1-Phenyl-3-methylamino-propoxy)-isoflavan.
28. 6-(1-Phenyl-3-dimethylamino-propoxy)-isoflavan.
29. 7-(1-Phenyl-3-dimethylamino-propoxy)-isoflavan, F. 98—100°.
30. 6-(1-Phenyl-3-N-benzyl-N-methylamino-propoxy)-isoflavan.
31. 7-(1-Phenyl-3-N-benzyl-N-methylamino-propoxy)-isoflavan.
32. 6-[α-(1-Methyl-3-piperidyl)-benzyloxy]-isoflavan.
33. 7-[α-(1-Methyl-3-piperidyl)-benzyloxy]-isoflavan.
34. 6-[2-(1-Methyl-2-piperidyl)-äthoxy]-isoflavan.
35. 7-[2-(1-Methyl-2-piperidyl)-äthoxy]-isoflavan.
36. 6-(1,3-Dimethyl-4-hexahydroazepinyloxy)-isoflavan.
37. 7-(1,3-Dimethyl-4-hexahydroazepinyloxy)-isoflavan.
38. 6-(1-Phenyl-3-methylamino-propoxy)-chromanon.
39. 7-(1-Phenyl-3-methylamino-propoxy)-chromanon.
40. 6-(1-Phenyl-3-dimethylamino-propoxy)-chromanon.
41. 7-(1-Phenyl-3-dimethylamino-propoxy)-chromanon, Hydrochlorid, F. 188—191°.
42. 6-(1-Phenyl-3-N-benzyl-N-methylamino-propoxy)-chromanon.
43. 7-(1-Phenyl-3-N-benzyl-N-methylamino-propoxy)-chromanon.
44. 6-[α-(1-Methyl-3-piperidyl)-benzyloxy]-chromanon.
45. 7-[α-(1-Methyl-3-piperidyl)-benzyloxy]-chromanon.
46. 6-[2-(1-Methyl-2-piperidyl)-äthoxy]-chromanon.
47. 7-[2-(1-Methyl-2-piperidyl)-äthoxy]-chromanon, Hydrochlorid, F. 182—184°.
48. 6-(1,3-Dimethyl-4-hexahydroazepinyloxy)-chromanon.
49. 7-(1,3-Dimethyl-4-hexahydroazepinyloxy)-chromanon.
50. 6-(1-Phenyl-3-methylamino-propoxy)-flavanon.
51. 7-(1-Phenyl-3-methylamino-propoxy)-flavanon.
52. 6-(1-Phenyl-3-dimethylamino-propoxy)-flavanon.
53. 7-(1-Phenyl-3-dimethylamino-propoxy)-flavanon.
54. 6-(1-Phenyl-3-N-benzyl-N-methylamino-propoxy)-flavanon.
55. 7-(1-Phenyl-3-N-benzyl-N-methylamino-propoxy)-flavanon.
56. 6-[α-(1-Methyl-3-piperidyl)-benzyloxy]-flavanon.
57. 7-[α-(1-Methyl-3-piperidyl)-benzyloxy]-flavanon.
58. 6-[2-(1-Methyl-2-piperidyl)-äthoxy]-flavanon.
59. 7-[2-(1-Methyl-2-piperidyl)-äthoxy]-flavanon.
60. 6-(1,3-Dimethyl-4-hexahydroazepinyloxy)-flavanon.
61. 7-(1,3-Dimethyl-4-hexahydroazepinyloxy)-flavanon.
62. 6-(1-Phenyl-3-methylamino-propoxy)-isoflavanon.
63. 7-(1-Phenyl-3-methylamino-propoxy)-isoflavanon.
64. 6-(1-Phenyl-3-dimethylamino-propoxy)-isoflavanon.
65. 7-(1-Phenyl-3-dimethylamino-propoxy)-isoflavanon.
66. 6-(1-Phenyl-3-N-benzyl-N-methylamino-propoxy)-isoflavanon.
67. 7-(1-Phenyl-3-N-benzyl-N-methylamino-propoxy)-isoflavanon.
68. 6-[α-(1-Methyl-3-piperidyl)-benzyloxy]-isoflavanon.
69. 7-[α-(1-Methyl-3-piperidyl)-benzyloxy]-isoflavanon.
70. 6-[2-(1-Methyl-2-piperidyl)-äthoxy]-isoflavanon.
71. 7-[2-(1-Methyl-2-piperidyl)-äthoxy]-isoflavanon.
72. 6-(1,3-Dimethyl-4-hexahydroazepinyloxy)-isoflavanon.
73. 7-(1,3-Dimethyl-4-hexahydroazepinyloxy)-isoflavanon.
74. 6-(1-Phenyl-3-methylamino-propoxy)-chromon.
75. 7-(1-Phenyl-3-methylamino-propoxy)-chromon.
76. 6-(1-Phenyl-3-dimethylamino-propoxy)-chromon.
77. 7-(1-Phenyl-3-dimethylamino-propoxy)-chromon, Hydrochlorid, F. 199°.
78. 6-(1-Phenyl-3-N-benzyl-N-methylamino-propoxy)-chromon.
79. 7-(1-Phenyl-3-N-benzyl-N-methylamino-propoxy)-chromon.
80. 6-[α-(1-Methyl-3-piperidyl)-benzyloxy]-chromon.

81. 7-[α-(1-Methyl-3-piperidyl)-benzyloxy]-chromon.
82. 6-[2-(1-Methyl-2-piperidyl)-äthoxy]-chromon.
83. 7-[2-(1-Methyl-2-piperidyl)-äthoxy]-chromon.
84. 6-(1,3-Dimethyl-4-hexahydroazepinyloxy)-chromon.
85. 6-(1,3-Dimethyl-4-hexahydroazepinyloxy)-chromon.
86. 5-(1-Phenyl-3-methylamino-propoxy)-flavon.
87. 6-(1-Phenyl-3-methylamino-propoxy)-flavon, Rf 0,43 (Methylenchlorid/Triäthylamin 8 : 2).
88. 7-(1-Phenyl-3-methylamino-propoxy)-flavon.
89. 5-(1-Phenyl-3-dimethylamino-propoxy)-flavon, F. 140—141°.
90. 6-(1-Phenyl-3-dimethylamino-propoxy)-flavon, F. 142—142,5°.
91. 8-(1-Phenyl-3-dimethylamino-propoxy)-flavon.
92. 5-(1-Phenyl-3-N-benzyl-N-methylamino-propoxy)-flavon.
93. 6-(1-Phenyl-3-N-benzyl-N-methylamino-propoxy)-flavon. F. 93—94°.
94. 7-(1-Phenyl-3-N-benzyl-N-methylamino-propoxy)-flavon.
95. 5-(3-Piperidyl-methoxy)-flavon.
96. 6-(3-Piperidyl-methoxy)-flavon.
97. 7-(3-Piperidyl-methoxy)-flavon.
98. 5-(α-3-Piperidyl-benzyloxy)-flavon.
99. 6-(α-3-Piperidyl-benzyloxy)-flavon.
100. 7-(α-3-Piperidyl-benzyloxy-flavon.
101. 5-(1-Methyl-3-piperidyl-methoxy)-flavon.
102. 6-(1-Methyl-3-piperidyl-methoxy)-flavon.
103 7-(1-Methyl-3-piperidyl-methoxy)-flavon.
104. 5-(α-1-Methyl-3-piperidyl-benzyloxy)-flavon.
105. 6-(α-1-Methyl-3-piperidyl-benzyloxy)-flavon.
106. 7-(α-1-Methyl-3-piperidyl-benzyloxy)-flavon.
107. 5-[2-(2-Piperidyl)-äthoxy]-flavon.
108. 6-[2-(2-Piperidyl)-äthoxy]-flavon.
109. 7-[2-(2-Piperidyl)-äthoxy]-flavon.
110. 5-[1-Phenyl-2-(2-piperidyl)-äthoxy]-flavon.
111. 6-[1-Phenyl-2-(2-piperidyl)-äthoxy]-flavon.
112. 7-[1-Phenyl-2-(2-piperidyl)-äthoxy]-flavon.
113. 5-[2-(1-Methyl-2-piperidyl)-äthoxy]-flavon.
114. 6-[2-(1-Methyl-2-piperidyl)-äthoxy]-flavon.
115. 7-[2-(1-Methyl-2-piperidyl)-äthoxy]-flavon.
116. 5-[1-Phenyl-2-(1-methyl-2-piperidyl)-äthoxy]-flavon.
117. 6-[1-Phenyl-2-(1-methyl-2-piperidyl)-äthoxy]-flavon.
118. 7-[1-Phenyl-2-(1-methyl-2-piperidyl)-äthoxy]-flavon.
119. 5-(4-Hexahydroazepinyloxy)-flavon.
120. 6-(4-Hexahydroazepinyloxy)-flavon.
121. 7-(4-Hexahydroazepinyloxy)-flavon.
121. 7-(4-Hexahydroazepinyloxy)-flavon.
122. 5-(1-Methyl-4-hexahydroazepinyloxy)-flavon.
123. 6-(1-Methyl-4-hexahydroazepinyloxy)-flavon.
124. 7-(1-Methyl-4-hexahydroazepinyloxy)-flavon.
125. 5-(3-Methyl-4-hexahydroazepinyloxy)-flavon.
126. 6-(3-Methyl-4-hexahydroazepinyloxy)-flavon.
127. 7-(3-Methyl-4-hexahydroazepinyloxy)-flavon.
128. 5-(1,3-Dimethyl-4-hexahydroazepinyloxy)-flavon.
129. 6-(1,3-Dimethyl-4-hexahydroazepinyloxy)-flavon.
130. 7-(1,3-Dimethyl-4-hexahydroazepinyloxy)-flavon.
131. 5-(1-Phenyl-3-methylamino-propoxy)-isoflavon.
132. 6-(1-Phenyl-3-methylamino-propoxy)-isoflavon.
133. 7-(1-Phenyl-3-methylamino-propoxy)-isoflavon, Hydrochlorid, F. 210—211°.
134. 5-(1-Phenyl-3-dimethylamino-propoxy)-isoflavon.
135. 6-(1-Phenyl-3-dimethylamino-propoxy)-isoflavon.
136. 8-(1-Phenyl-3-dimethylamino-propoxy)-isoflavon.
137. 5-(1-Phenyl-3-N-benzyl-N-methylamino-propoxy)-isoflavon.
138. 6-(1-Phenyl-3-N-benzyl-N-methylamino-propoxy)-isoflavon.
139. 7-(1-Phenyl-3-N-benzyl-N-methylamino-propoxy)-isoflavon, F. 115—118°, Hydrochlorid, F. 210—212°.
140. 5-(3-Piperidyl-methoxy)-isoflavon.
141. 6-(3-Piperidyl-methoxy)-isoflavon.
142. 7-(3-Piperidyl-methoxy)-isoflavon.
143. 5-(α-3-Piperidyl-benzyloxy)-isoflavon.

144. 6-(α-3-Piperidyl-benzyloxy)-isoflavon.
145. 7-(α-3-Piperidyl-benzyloxy)-isoflavon.
146. 5-(1-Methyl-3-piperidyl-methoxy)-isoflavon.
147. 6-(1-Methyl-3-piperidyl-methoxy)-isoflavon.
148. 7-(1-Methyl-3-piperidyl-methoxy)-isoflavon, Hydrochlorid, F. 257–260°.
149. 5-(α-1-Methyl-3-piperidyl-benzyloxy)-isoflavon.
150. 6-(α-1-Methyl-3-piperidyl-benzyloxy)-isoflavon.
151. 7-(α-1-Methyl-3-piperidyl-benzyloxy)-isoflavon, Hydrochlorid, F. 143–145°.
152. 5-[2-(2-Piperidyl)-äthoxy]-isoflavon.
153. 6-[2-(2-Piperidyl)-äthoxy]-isoflavon.
154. 7-[2-(2-Piperidyl)-äthoxy]-isoflavon.
155. 5-[1-Phenyl-2-(2-piperidyl)-äthoxy]-isoflavon.
156. 6-[1-Phenyl-2-(2-piperidyl)-äthoxy]-isoflavon.
157. 7-[1-Phenyl-2-(2-piperidyl)-äthoxy]-isoflavon.
158. 5-[2-(1-Methyl-2-piperidyl)-äthoxy]-isoflavon.
159. 6-[2-(1-Methyl-2-piperidyl)-äthoxy]-isoflavon.
160. 7-[2-(1-Methyl-2-piperidyl)-äthoxy]-isoflavon, Hydrochlorid, F. 206–207°.
161. 5-[1-Phenyl-2-(1-methyl-2-piperidyl)-äthoxy]-isoflavon.
162. 6-[1-Phenyl-2-(1-methyl-2-piperidyl)-äthoxy]-isoflavon.
163. 7-[1-Phenyl-2-(1-methyl-2-piperidyl)-äthoxy]-isoflavon.
164. 5-(4-Hexahydroazepinyloxy)-isoflavon.
165. 6-(4-Hexahydroazepinyloxy)-isoflavon.
166. 7-(4-Hexahydroazepinyloxy)-isoflavon.
167. 5-(1-Methyl-4-hexahydroazepinyloxy)-isoflavon.
168. 6-(1-Methyl-4-hexahydroazepinyloxy)-isoflavon.
169. 7-(1-Methyl-4-hexahydroazepinyloxy)-isoflavon.
170. 5-(3-Methyl-4-hexahydroazepinyloxy)-isoflavon.
171. 6-(3-Methyl-4-hexahydroazepinyloxy)-isoflavon.
172. 7-(3-Methyl-4-hexahydroazepinyloxy)-isoflavon.
173. 5-(1,3-Dimethyl-4-hexahydroazepinyloxy)-isoflavon.
174. 6-(1,3-Dimethyl-4-hexahydroazepinyloxy)-isoflavon.
175. 7-(1,3-Dimethyl-4-hexahydroazepinyloxy)-isoflavon.
176. 5-(1-Phenyl-3-methylamino-propoxy)-1-tetralon.
177. 6-(1-Phenyl-3-methylamino-propoxy)-1-tetralon.
178. 7-(1-Phenyl-3-methylamino-propoxy)-1-tetralon.
179. 5-(1-Phenyl-3-dimethylamino-propoxy)-1-tetralon.
180. 6-(1-Phenyl-3-dimethylamino-propoxy)-1-tetralon.
181. 7-(1-Phenyl-3-dimethylamino-propoxy)-1-tetralon, Hydrochlorid, F. 167–169°.
182. 5-(1-Phenyl-3-N-benzyl-N-methylamino-propoxy)-1-tetralon.
183. 6-(1-Phenyl-3-N-benzyl-N-methylamino-propoxy)-1-tetralon, Rf 0,4 (Äthylacetat).
184. 7-(1-Phenyl-3-N-benzyl-N-methylamino-propoxy)-1-tetralon, Hydrochlorid, F. 178–180°.
185. 5-(3-Piperidyl-methoxy)-1-tetralon.
186. 6-(3-Piperidyl-methoxy)-1-tetralon.
187. 7-(3-Piperidyl-methoxy)-1-tetralon.
188. 5-(α-3-Piperidyl-benzyloxy)-1-tetralon.
189. 6-(α-3-Piperidyl-benzyloxy)-1-tetralon.
190. 7-(α-3-Piperidyl-benzyloxy)-1-tetralon.
191. 5-(1-Methyl-3-piperidyl-methoxy)-1-tetralon.
192. 6-(1-Methyl-3-piperidyl-methoxy)-1-tetralon.
193. 7-(1-Methyl-3-piperidyl-methoxy)-1-tetralon.
194. 5-(α-1-Methyl-3-piperidyl-benzyloxy)-1-tetralon.
195. 6-(α-1-Methyl-3-piperidyl-benzyloxy)-1-tetralon.
196. 7-(α-1-Methyl-3-piperidyl-benzyloxy)-1-tetralon.
197. 5-[2-(2-Piperidyl)-äthoxy]-1-tetralon.
198. 6-[2-(2-Piperidyl)-äthoxy]-1-tetralon.
199. 7-[2-(2-Piperidyl)-äthoxy]-1-tetralon.
200. 5-[1-Phenyl-2-(2-piperidyl)-äthoxy]-1-tetralon.
201. 6-[1-Phenyl-2-(2-piperidyl)-äthoxy]-1-tetralon.
202. 7-[1-Phenyl-2-(2-piperidyl)-äthoxy]-1-tetralon.
203. 5-[2-(1-Methyl-2-piperidyl)-äthoxy]-1-tetralon.
204. 6-[2-(1-Methyl-2-piperidyl)-äthoxy]-1-tetralon.
205. 7-[2-(1-Methyl-2-piperidyl)-äthoxy]-1-tetralon, Hydrochlorid, F. 188–190°.
206. 5-[1-Phenyl-2-(1-methyl-2-piperidyl)-äthoxy]-1-tetralon.
207. 6-[1-Phenyl-2-(1-methyl-2-piperidyl)-äthoxy]-1-tetralon.
208. 7-[1-Phenyl-2-(1-methyl-2-piperidyl)-äthoxy]-1-tetralon.

209. 5-(4-Hexahydroazepinyloxy)-1-tetralon.
210. 6-(4-Hexahydroazepinyloxy)-1-tetralon.
211. 7-(4-Hexahydroazepinyloxy)-1-tetralon.
212. 5-(1-Methyl-4-hexahydroazepinyloxy)-1-tetralon.
213. 6-(1-Methyl-4-hexahydroazepinyloxy)-1-tetralon.
214. 7-(1-Methyl-4-hexahydroazepinyloxy)-1-tetralon.
215. 5-(3-Methyl-4-hexahydroazepinyloxy)-1-tetralon.
216. 6-(3-Methyl-4-hexahydroazepinyloxy)-1-tetralon.
217. 7-(3-Methyl-4-hexahydroazepinyloxy)-1-tetralon.
218. 5-(1,3-Dimethyl-4-hexahydroazepinyloxy)-1-tetralon.
219. 6-(1,3-Dimethyl-4-hexahydroazepinyloxy)-1-tetralon.
220. 7-(1,3-Dimethyl-4-hexahydroazepinyloxy)-1-tetralon.

Beispiele 221 bis 313

Analog Beispiel 1 erhält man aus den entsprechenden Na-Phenolaten und den entsprechenden Chlor- oder Brom-aminen:

221. 6-(1-Cyclopropyl-3-dimethylamino-propoxy)-isoflavon.
222. 7-(1-Cyclopropyl-3-dimethylamino-propoxy)-isoflavon.
223. 6-(1-p-Fluorphenyl-3-dimethylamino-propoxy)-isoflavon.
224. 7-(1-p-Fluorphenyl-3-dimethylamino-propoxy)-isoflavon, Hydrochlorid, F. 217°.
225. 6-(1-p-Chlorphenyl-3-dimethylamino-propoxy)-isoflavon.
226. 7-(1-p-Chlorphenyl-3-dimethylamino-propoxy)-isoflavon, Hydrochlorid, F. 247°.
227. 6-(1-p-Methoxyphenyl-3-dimethylamino-propoxy)-isoflavon.
228. 7-(1-p-Methoxyphenyl-3-dimethylamino-propoxy)-isoflavon, F. 111–113°.
229. 6-(1-p-Methylthiophenyl-3-dimethylamino-propoxy)-isoflavon.
230. 7-(1-p-Methylthiophenyl-3-dimethylamino-propoxy)-isoflavon.
231. 6-(1-Phenyl-3-dimethylamino-propoxy)-4'-methoxy-flavan, Hydrochlorid, F. 166–168°.
232. 7-(1-Phenyl-3-dimethylamino-propoxy)-4'-methoxy-flavan.
233. 3-Methyl-6-(1-phenyl-3-dimethylamino-propoxy)-3',4'-methylendioxy-flavanon, Hydrochlorid, F. 80–120°.
234. 3-Methyl-7-(1-phenyl-3-dimethylamino-propoxy)-3',4'-methylendioxy-flavanon.
235. cis-3-Äthyl-6-(1-phenyl-3-dimethylamino-propoxy)-4'-methoxy-flavanon, Rf 0,55.
236. trans-3-Äthyl-6-(1-phenyl-3-dimethylamino-propoxy)-4'-methoxy-flavanon, Rf 0,57.
237. cis-3-n-Propyl-6-(1-phenyl-3-dimethylamino-propoxy)-flavanon, Rf 0,44.
238. 3-tert.-Butyl-6-(1-phenyl-3-methylamino-propoxy)-chromon.
239. 3-tert.-Butyl-7-(1-phenyl-3-methylamino-propoxy)-chromon.
240. 3-tert.-Butyl-6-(1-phenyl-3-dimethylamino-propoxy)-chromon.
241. 3-tert.-Butyl-7-(1-phenyl-3-dimethylamino-propoxy)-chromon.
242. 3-tert.-Butyl-6-(1-phenyl-3-N-benzyl-N-methylamino-propoxy)-chromon.
243. 3-tert.-Butyl-7-(1-phenyl-3-N-benzyl-N-methylamino-propoxy)-chromon.
244. 3-tert.-Butyl-6-[α-(1-methyl-3-piperidyl)-benzyloxy]-chromon.
245. 3-tert.-Butyl-7-[α-(1-methyl-3-piperidyl)-benzyloxy]-chromon.
246. 3-tert.-Butyl-6-[2-(1-methyl-2-piperidyl)-äthoxy]-chromon.
247. 3-tert.-Butyl-7-[2-(1-methyl-2-piperidyl)-äthoxy]-chromon.
248. 3-tert.-Butyl-6-(1,3-dimethyl-4-hexahydroazepinyloxy)-chromon.
249. 3-tert.-Butyl-7-(1,3-dimethyl-4-hexahydroazepinyloxy)-chromon.
250. 2-Cyclopentyl-6-(1-phenyl-3-methylamino-propoxy)-chromon.
251. 2-Cyclopentyl-7-(1-phenyl-3-methylamino-propoxy)-chromon.
252. 2-Cyclopentyl-6-(1-phenyl-3-dimethylamino-propoxy)-chromon.
253. 2-Cyclopentyl-7-(1-phenyl-3-dimethylamino-propoxy)-chromon.
254. 2-Cyclopentyl-6-(1-phenyl-3-N-benzyl-N-methylamino-propoxy)-chromon.
255. 2-Cyclopentyl-7-(1-phenyl-3-N-benzyl-N-methylamino-propoxy)-chromon.
256. 2-Cyclopentyl-6-[α-(1-methyl-3-piperidyl)-benzyloxy]-chromon.
257. 2-Cyclopentyl-7-[α-(1-methyl-3-piperidyl)-benzyloxy]-chromon.
258. 2-Cyclopentyl-6-[2-(1-methyl-2-piperidyl)-äthoxy]-chromon.
259. 2-Cyclopentyl-7-[2-(1-methyl-2-piperidyl)-äthoxy]-chromon.
260. 2-Cyclopentyl-6-(1,3-dimethyl-4-hexahydroazepinyloxy)-chromon.
261. 2-Cyclopentyl-7-(1,3-dimethyl-4-hexahydroazepinyloxy)-chromon.
262. 3-Cyclopentyl-6-(1-phenyl-3-methylamino-propoxy)-chromon.
263. 3-Cyclopentyl-7-(1-phenyl-3-methylamino-propoxy)-chromon.
264. 3-Cyclopentyl-6-(1-phenyl-3-dimethylamino-propoxy)-chromon.
265. 3-Cyclopentyl-7-(1-phenyl-3-dimethylamino-propoxy)-chromon.

266. 3-Cyclopentyl-6-(1-phenyl-3-N-benzyl-N-methylamino-propoxy)-chromon.
267. 3-Cyclopentyl-7-(1-phenyl-3-N-benzyl-N-methylamino-propoxy)-chromon.
268. 3-Cyclopentyl-6-[α-1-(methyl-3-piperidyl)-benzyloxy]-chromon.
269. 3-Cyclopentyl-7-[α-1-(methyl-3-piperidyl)-benzyloxy]-chromon.
270. 3-Cyclopentyl-6-[2-(1-methyl-2-piperidyl)-äthoxy]-chromon.
271. 3-Cyclopentyl-7-[2-(1-methyl-2-piperidyl)-äthoxy]-chromon.
272. 3-Cyclopentyl-6-(1,3-dimethyl-4-hexahydroazepinyloxy)-chromon.
273. 3-Cyclopentyl-7-(1,3-dimethyl-4-hexahydroazepinyloxy)-chromon.
274. 2-Cyclohexyl-6-(1-phenyl-3-methylamino-propoxy)-chromon.
275. 2-Cyclohexyl-7-(1-phenyl-3-methylamino-propoxy)-chromon.
276. 2-Cyclohexyl-6-(1-phenyl-3-dimethylamino-propoxy)-chromon.
277. 2-Cyclohexyl-7-(1-phenyl-3-dimethylamino-propoxy)-chromon.
278. 2-Cyclohexyl-6-(1-phenyl-3-N-benzyl-N-methylamino-propoxy)-chromon.
279. 2-Cyclohexyl-7-(1-phenyl-3-N-benzyl-N-methylamino-propoxy)-chromon.
280. 2-Cyclohexyl-6-[α-(1-methyl-3-piperidyl)-benzyloxy]-chromon.
281. 2-Cyclohexyl-7-[α-(1-methyl-3-piperidyl)-benzyloxy]-chromon.
282. 2-Cyclohexyl-6-[2-(1-methyl-2-piperidyl)-äthoxy]-chromon.
283. 2-Cyclohexyl-7-[2-(1-methyl-2-piperidyl)-äthoxy]-chromon.
284. 2-Cyclohexyl-6-(1,3-dimethyl-4-hexahydroazepinyloxy)-chromon.
285. 2-Cyclohexyl-7-(1,3-dimethyl-4-hexahydroazepinyloxy)-chromon.
286. 3-Cyclohexyl-6-(1-phenyl-3-methylamino-propoxy)-chromon.
287. 3-Cyclohexyl-7-(1-phenyl-3-methylamino-propoxy)-chromon.
288. 3-Cyclohexyl-6-(1-phenyl-3-dimethylamino-propoxy)-chromon.
289. 3-Cyclohexyl-7-(1-phenyl-3-dimethylamino-propoxy)-chromon.
290. 3-Cyclohexyl-6-(1-phenyl-3-N-benzyl-N-methylamino-propoxy)-chromon.
291. 3-Cyclohexyl-7-(1-phenyl-3-N-benzyl-N-methylamino-propoxy)-chromon.
292. 3-Cyclohexyl-6-[α-(1-methyl-3-piperidyl)-benzyloxy]-chromon.
293. 3-Cyclohexyl-7-[α-(1-methyl-3-piperidyl)-benzyloxy]-chromon.
294. 3-Cyclohexyl-6-[2-(1-methyl-2-piperidyl)-äthoxy]-chromon.
295. 3-Cyclohexyl-7-[2-(1-methyl-2-piperidyl)-äthoxy]-chromon.
296. 3-Cyclohexyl-6-(1,3-dimethyl-4-hexahydroazepinyloxy)-chromon.
297. 3-Cyclohexyl-7-(1,3-dimethyl-4-hexahydroazepinyloxy)-chromon.
298. 3-Methyl-6-(1-phenyl-3-dimethylamino-propoxy)-3',4'-methylendioxy-flavon, F. 74—76°.
299. 3-Methyl-7-(1-phenyl-3-dimethylamino-propoxy)-3',4'-methylendioxy-flavon.
300. 2-Äthyl-6-(1-phenyl-3-dimethylamino-propoxy)-isoflavon
301. 2-Äthyl-7-(1-phenyl-3-dimethylamino-propoxy)-isoflavon, Hydrochlorid, F. 226—228°.
302. 6-(1-Phenyl-3-dimethylamino-propoxy)-4'-fluor-isoflavon.
303. 7-(1-Phenyl-3-dimethylamino-propoxy)-4'-fluor-isoflavon, F. 112—113°; Hydrochlorid, F. 234—235°.
304. 6-(1-Phenyl-3-dimethylamino-propoxy)-4'-chlor-isoflavon.
305. 7-(1-Phenyl-3-dimethylamino-propoxy)-4'-chlor-isoflavon, Hydrochlorid, F. 259—260°.
306. 6-(1-Phenyl-3-dimethylamino-propoxy)-3'-trifluormethyl-isoflavon.
307. 7-(1-Phenyl-3-dimethylamino-propoxy)-3'-trifluormethyl-isoflavon, Hydrochlorid, F. 174—176°.
308. 6-(1-p-Fluorphenyl-3-dimethylamino-propoxy)-4'-fluor-isoflavon.
309. 7-(1-p-Fluorphenyl-3-dimethylamino-propoxy)-4'-fluor-isoflavon, F. 121—123°, Hydrochlorid, F. 229—230°.
310. 6-(1-p-Fluorphenyl-3-dimethylamino-propoxy)-4'-chlor-isoflavon.
311. 7-(1-p-Fluorphenyl-3-dimethylamino-propoxy)-4'-chlor-isoflavon, Hydrochlorid, F. 213—215°.
312. 6-(1-p-Fluorphenyl-3-dimethylamino-propoxy)-3'-trifluormethyl-isoflavon.
313. 7-(1-p-Fluorphenyl-3-dimethylamino-propoxy)-3'-trifluormethyl-isoflavon, Hydrochlorid, F. 174—176°.

## Beispiel 314

Man löst 23,8 g 7-Hydroxyisoflavon in 500 ml absolutem Toluol, versetzt mit 7,1 ml Thallium-(I)-äthylat und rührt 1 Stunde bei 20°. Nach dem Eindampfen wird der Rückstand in 100 ml absolutem Acetonitril gelöst, mit 20 g 1-Chlor-1-phenyl-3-dimethylaminopropan versetzt, 3 Stunden unter Rühren gekocht und eingedampft. Nach üblicher Aufarbeitung erhält man 7-(1-Phenyl-3-dimethylamino-propoxy)-isoflavon. F. 128—130°.

Beispiel 315

Man löst 2,7 g Azodicarbonsäureäthylester in 25 ml THF, gibt unter Kühlung und Rühren 4 g Triphenylphosphin hinzu, tropft anschließend eine Lösung von 2 g 1-Chlor-1-phenyl-3-dimethylamino-propan in 15 ml THF und dann eine Lösung von 2,38 g 7-Hydroxyisoflavon in 10 ml THF hinzu und rührt 2 Stunden bei 0°. Nach Stehenlassen über Nacht bei 20° wird wie üblich aufgearbeitet. Man erhält 7-(1-Phenyl-3-dimethylamino-propoxy)-isoflavon. F. 128—130°.

Beispiel 316

Zu einer Lösung von 23,8 g 7-Hydroxyisoflavon in 100 ml $CH_2Cl_2$ gibt man 200 ml 50%ige wässerige Natronlauge und 1 g Triäthylbenzylammoniumchlorid, tropft unter Rühren 20 g 1-Chlor-1-phenyl-3-dimethylamino-propan hinzu und rührt noch eine Stunde nach. Nach üblicher Aufarbeitung erhält man 7-(1-Phenyl-3-dimethylamino-propoxy)-isoflavon. F. 128—130°.

Beispiel 317

Eine Lösung von 20 g 7-(1-Phenyl-3-N-benzyl-N-methylamino-propoxy)-isoflavon in 400 ml Methanol wird an 3 g 5%igem Pd—C bei 20° und 1 at bis zum Stillstand hydriert. Man filtriert, dampft ein und erhält 7-(1-Phenyl-3-methylamino-propoxy)-isoflavon, Hydrochlorid, F. 210—211°.

Beispiel 318

Zu 38,5 g 7-(1-Phenyl-3-methylamino-propoxy)-isoflavon tropft man unter Rühren und Kühlen 30 g Ameisensäure und anschließend bei 20° 7 g 25%ige Formaldehydlösung. Man erhitzt bis zum Ende der Gasentwicklung auf dem Wasserbad, kühlt ab, gießt auf Eis, arbeitet wie üblich auf und erhält 7-(1-Phenyl-3-dimethylamino-propoxy)-isoflavon. F. 128—130°.

Beispiel 319

Man gibt 12 g Allylbromid und 26 g wasserfreies Kaliumcarbonat zu einer Lösung von 38,5 g 7-(1-Phenyl-3-methyl-amino-propoxy)-isoflavon in 1 l absolutem Toluol, kocht 20 Stunden, kühlt ab, gießt in Wasser, arbeitet wie üblich auf und erhält 7-(1-Phenyl-3-N-allyl-N-methylaminopropoxy)-isoflavon. Hydrochlorid, F. 192—194°.

Beispiele 320 bis 326

Analog Beispiel 319 erhält man mit Allylbromid, n-Butyljodid, Cyclopropylmethylchlorid oder Benzylbromid:

320. 6-(1-Phenyl-3-N-allyl-N-methylamino-propoxy)-isoflavon.
321. 6-(1-Phenyl-3-N-n-butyl-N-methylamino-propoxy)-isoflavon.
322. 7-(1-Phenyl-3-N-n-butyl-N-methylamino-propoxy)-isoflavon.
323. 6-(1-Phenyl-3-N-cyclopropylmethyl-N-methylamino-propoxy)-isoflavon.
324. 7-(1-Phenyl-3-N-cyclopropylmethyl-N-methylamino-propoxy)-isoflavon, Hydrochlorid, F. 225—227°.
325. 6-(1-Phenyl-3-N-benzyl-N-methylamino-propoxy)-isoflavon.
326. 7-(1-Phenyl-3-N-benzyl-N-methylamino-propoxy)-isoflavon.

Beispiele 327 bis 331

Analog Beispiel 1 erhält man aus den entsprechenden Na-phenolaten und den entsprechenden basischen Chloriden:

327. 6-(1-Cyclopropyl-3-methylamino-propoxy)-flavon, Rf 0,45 (Chloroform/Triäthylamin 8 : 2).
328. 6-(1-Phenyl-3-dimethylamino-propoxy)-4'-methoxy-flavon, F. 125—127°.
329. 6-(1-Phenyl-3-dimethylamino-propoxy)-3',4'-methylendioxy-flavon, F. 159—160°.
330. 7-(1-Cyclopropyl-3-methylamino-propoxy)-chroman, Rf 0,24 (Methanol).
331. 7-(1-Cyclopropyl-3-dimethylamino-propoxy)-chroman, Rf 0,74 (Chloroform/Triäthylamin 8 : 2).

Die nachstehenden Beispiele betreffen pharmazeutische Zubereitungen, die Amine der Formel I oder ihre Säureadditionssalze enthalten:

Beispiel A

Tabletten

Ein Gemisch von 1 kg 7-(1-Phenyl-3-dimethylamino-propoxy)-isoflavon-hydrochlorid, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 10 mg Wirkstoff enthält.

Beispiel B

Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C

Kapseln

2 kg 7-(1-Phenyl-3-dimethylamino-propoxy)-isoflavon-hydrochlorid werden in üblicher Weise in Hartgelatinekapseln gefüllt, so daß jede Kapsel 20 mg des Wirkstoffs enthält.

Beispiel D

Ampullen

Eine Lösung von 1 kg 7-(1-Phenyl-3-dimethylamino-propoxy)-isoflavon-hydrochorid in 30 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffen der Formel I und/oder ihre physiologisch unbedenklichen Säureadditionssalze enthalten.

**Patentansprüche**

1. Basische Äther der allgemeinen Formel I

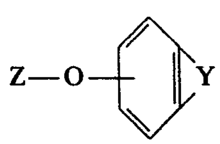

(I)

worin

| | |
|---|---|
| Z | $CH_3-NR^2-CH_2CH_2-CHR^1-$, $(1\text{-}R^2\text{-}3\text{-piperidyl})-CHR^3-$, $(1\text{-}R^2\text{-}2\text{-piperidyl})-CH_2-CHR^3-$ oder $1\text{-}R^2\text{-}3\text{-}R^4-4$-hexahydroazepinyl, |
| $R^1$ | Cyclopropyl oder Ar, |
| $R^2$ | H, Alkyl mit 1–4 C-Atomen, Allyl, Cycloalkylalkyl mit 4–8 C-Atomen oder Benzyl, |
| $R^3$ | H oder Ar, |
| $R^4$ | H oder Alkyl mit 1–4 C-Atomen, |
| Y | $-O-CHQ^1-CHQ^2-CH_2-$, $-O-CHQ^1-CHQ^2-CO-$, $-O-CQ^1=CQ^2-CO-$ oder $-CH_2-CHQ^1-CHQ^2-CO-$, |
| $Q^1$ und $Q^2$ | jeweils H, Alkyl mit 1–4 C-Atomen, Cycloalkyl mit 3–6 C-Atomen oder Ar und |
| Ar | Phenyl oder durch F, Cl, Alkoxy oder Alkylthio mit jeweils 1–4 C-Atomen, Methylendioxy oder $CF_3$ substituiertes Phenyl |

bedeuten, worin jedoch

$R^1$ Cyclopropyl oder durch F, Cl, Alkoxy oder Alkylthio mit jeweils 1—4 C-Atomen, Methylendioxy oder $CF_3$ substituiertes Phenyl

bedeutet, wenn gleichzeitig

Z eine $(CH_3)_2N-CH_2CH_2-CHR^1$-Gruppe in 7-Stellung und
Y $-O-C(C_6H_5)=CH-CO-$ oder $-O-C(C_6H_5)=C(CH_3)-CO-$

bedeuten, sowie ihre physiologisch unbedenklichen Säureadditionssalze.

2. 6-(1-Phenyl-3-dimethylamino-propoxy)-flavon.

3. Verfahren zur Herstellung von basischen Äthern der allgemeinen Formel I

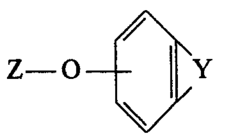

(I)

worin

Z $CH_3-NR^2-CH_2CH_2-CHR^1-$, $(1\text{-}R^2\text{-}3\text{-piperidyl})-CHR^3-$, $(1\text{-}R^2\text{-}2\text{-piperidyl})-CH_2-CHR^3-$ oder $1\text{-}R^2\text{-}3\text{-}R^4-4$-hexahydroazepinyl,
$R^1$ Cyclopropyl oder Ar,
$R^2$ H, Alkyl mit 1—4 C-Atomen, Allyl, Cycloalkylalkyl mit 4—8 C-Atomen oder Benzyl,
$R^3$ H oder Ar,
$R^4$ H oder Alkyl mit 1—4 C-Atomen,
Y $-O-CHQ^1-CHQ^2-CH_2-$, $-O-CHQ^1-CHQ^2-CO-$, $-O-CQ^1=CQ^2-CO-$ oder $-CH_2-CHQ^1-CHQ^2-CO-$,
$Q^1$ und $Q^2$ jeweils H, Alkyl mit 1—4 C-Atomen, Cycloalkyl mit 3—6 C-Atomen oder Ar und
Ar Phenyl oder durch F, Cl, Alkoxy oder Alkylthio mit jeweils 1—4 C-Atomen, Methylendioxy oder $CF_3$ substituiertes Phenyl

bedeuten, worin jedoch

$R^1$ Cyclopropyl oder durch F, Cl, Alkoxy oder Alkylthio mit jeweils 1—4 C-Atomen, Methylendioxy oder $CF_3$ substituiertes Phenyl

bedeutet, wenn gleichzeitig

Z eine $(CH_3)_2N-CH_2CH_2-CHR^1$-Gruppe in 7-Stellung und
Y $-O-C(C_6H_5)=CH-CO-$ oder $-O-C(C_6H_5)=C(CH_3)-CO-$

bedeuten, sowie von deren physiologisch unbedenklichen Säureadditionssalzen, dadurch gekennzeichnet, daß man ein Phenol der allgemeinen Formel II

$$HO-G \quad (II)$$

worin
G die Gruppe

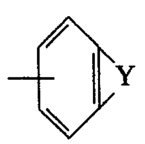

bedeutet, oder eines seiner Salze mit einem Amin der allgemeinen Formel III

$$Z-X \quad (III)$$

worin

X Cl, Br, J oder OH bedeutet und
Z die angegebene Bedeutung hat

oder mit einem seiner reaktionsfähigen Derivate umsetzt und daß man gegebenenfalls in einer erhaltenen Verbindung der Formel I eine sekundäre Aminogruppe durch Behandeln mit einem alkylierenden, allylierenden, cycloalkylalkylierenden oder benzylierenden Mittel in die entsprechende tertiäre Aminogruppe oder eine N-Benzylgruppe durch Behandeln mit einem Reduktionsmittel in eine NH-Gruppe und/oder eine erhaltene Base der Formel I durch Behandeln mit einer Säure in eines ihrer physiologisch unbedenklichen Säureadditionssalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Säureadditionssalze gemäß Anspruch 1 zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

5. Pharmazeutische Zubereitung, gekennzeichnet durch einen Gehalt an mindestens einer Verbindung der allgemeinen Formel I und/oder einem ihrer physiologisch unbedenklichen Säureadditionssalze gemäß Anspruch 1.

**Claims**

1. Basic ethers of the general formula I

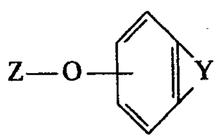

(I)

wherein

Z is $CH_3-NR^2-CH_2CH_2-CHR^1-$, (1-$R^2$-3-piperidyl)$-CHR^3-$, (1-$R^2$-2-piperidyl)$-CH_2CHR^3-$ or 1-$R^2$-3-$R^4$-4-hexahydroazepinyl,
$R^1$ is cyclopropyl or Ar,
$R^2$ is H, alkyl having 1–4 C atoms, alkenyl having 2–4 C atoms, cycloalkylalkyl having 4–8 C atoms or benzyl,
$R^3$ is H or Ar,
$R^4$ is H or alkyl having 1–4 c atoms,
Y is $-O-CHQ^1-CHQ^2-CH_2-$, $-O-CHQ^1-CHQ^2-CO-$, $-O-CQ^1{=}CQ^2-CO-$ or $-CH_2-CHQ^1-CHQ^2-CO-$,
$Q^1$ and $Q^2$ are each H, alkyl having 1–4 C atoms, cycloalkyl having 3–6 C atoms or Ar and
Ar is phenyl or phenyl which is substituted by F, Cl, alkoxy or alkylthio, each of which has 1–4 C atoms, methylenedioxy or $CF_3$,

but wherein

$R^1$ is cyclopropyl or phenyl which is substituted by F, Cl, alkoxy or alkylthio, each of which has 1–4 C atoms, methylenedioxy or $CF_3$,

if, at the same time,

Z is a $(CH_3)_2N-CH_2CH_2-CHR^1$ group in 7-position and
Y is a $-O-C(C_6H_5){=}CH-CO-$ or $-O-C(C_6H_5){=}C(CH_3)-CO$-group

and also their physiologically acceptable acid addition salts.

2. 6-(1-Phenyl-3-dimethylaminopropoxy)-flavone.

3. Process for the preparation of basic ethers of the general formula I

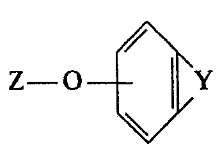

(I)

wherein

Z is $CH_3-NR^2-CH_2CH_2-CHR^1-$, (1-$R^2$-3-piperidyl)$-CHR^3-$, (1-$R^2$-2-piperidyl)$-CH_2CHR^3-$ or 1-$R^2$-3-$R^4$-4-hexahydroazepinyl,

| | |
|---|---|
| $R^1$ | is cyclopropyl or Ar, |
| $R^2$ | is H, alkyl having 1–4 C atoms, alkenyl having 2–4 C atoms, cycloalkylalkyl having 4–8 C atoms or benzyl, |
| $R^3$ | is H or Ar, |
| $R^4$ | is H or alkyl having 1–4 c atoms, |
| Y | is $-O-CHQ^1-CHQ^2-CH_2-$, $-O-CHQ^1-CHQ^2-CO-$, $-O-CQ^1{=}CQ^2-CO-$ or $-CH_2-CHQ^1-CHQ^2-CO-$, |
| $Q^1$ and $Q^2$ | are each H, alkyl having 1–4 C atoms, cycloalkyl having 3–6 C atoms or Ar and |
| Ar | is phenyl or phenyl which is substituted by F, Cl, alkoxy or alkylthio, each of which has 1–4 C atoms, methylenedioxy or $CF_3$, |

but wherein

$R^1$ is cyclopropyl or phenyl which is substituted by F, Cl, alkoxy or alkylthio, each of which has 1–4 C atoms, methylenedioxy or $CF_3$,

if, at the same time,

Z is a $(CH_3)_2N-CH_2CH_2-CHR^1$ group in 7-position and
Y is a $-O-C(C_6H_5){=}CH-CO-$ or $-O-C(C_6H_5){=}C(CH_3)-CO$-group,

and of their physiologically acceptable acid addition salts charakterised in that a phenol of the general formula II

HO—G (II)

wherein
G is the group

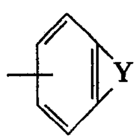

or one of its salts, is reacted with an amine of the general formula III

Z—X (III)

wherein

X is Cl, Br, I or OH and
Z has the meaning indicated,

or with one of its reactive derivatives, and, if appropriate, in a resulting compound of the formula I, a secondary amino group is converted by treatment with an alkylating, alkenylating, cycloalkylalkylating or benzylating agent into the corresponding tertiary amino group or an N-benzyl group is converted by treatment with a reducing agent into an NH group and/or a resulting base of the formula I is converted by treatment with an acid into one of its physiologically acceptable acid addition salts.

4. Process for the preparation of pharmaceutical formulations, characterised in that a compound of the formula I and/or one of its physiologically acceptable acid addition salts is brought into a suitable dosage form together with at least one solid, liquid or semi-liquid excipient or adjuvant, and, if appropriate, in combination with one or more further active compounds.

5. Pharmaceutical formulation characterised in that it contains at least one compound of the general formula I and/or one of its physiologically acceptable acid additions salts.

**Revendications**

1. Ethers basiques de formule générale I:

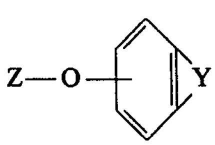

(I)

dans laquelle

Z représente $CH_3-NR^2-CH_2CH_2-CHR^1$, (1-$R^2$-3-pipéridyl)$-CHR^3-$, (1-$R^2$-2-pipéridyl)$-CH_2-CHR^3-$ ou 1-$R^2$-3-$R^4$-4-hexahydroazépinyle,
$R^1$ représente un groupe cyclopropyle ou Ar,
$R^2$ représente H, un groupe alkyle contenant 1 à 4 atomes de carbone, un groupe allyle, un groupe cycloalkylalkyle contenant 4 à 8 atomes de carbone ou un groupe benzyle,
$R^3$ représente H ou Ar,
$R^4$ représente H ou un groupe alkyle contenant 1 à 4 atomes de carbone,
Y représente $-O-CHQ^1-CHQ^2-CH_2-$, $-O-CHQ^1-CHQ^2-CO-$, $-O-CQ^1{=}CQ^2-CO-$ ou $-CH_2-CHQ^1-CHQ^2-CO-$,
$Q^1$ et $Q^2$ représentent chacun H, un groupe alkyle contenant 1 à 4 atomes de carbone, un groupe cycloalkyle contenant 3 à 6 atomes de carbone ou Ar, et
Ar représente un groupe phényle ou un groupe phényle substitué par F, Cl, un groupe alcoxy ou un groupe alkylthio contenant chacun 1 à 4 atomes de carbone, un groupe méthylène-dioxy ou par $CF_3$,

mais dans laquelle

$R^1$ représente un groupe cyclopropyle ou un groupe phényle substitué par F, Cl, un groupe alcoxy ou un groupe alkylthio contenant chacun 1 à 4 atomes de carbone, un groupe méthylène-dioxy ou par $CF_3$,

lorsque simultanément

Z représente un groupe $(CH_3)_2N-CH_2CH_2-CHR^1$ en position 7 et
Y représente $-O-C(C_6H_5){=}CH-CO$ ou $-O-C(C_6H_5){=}C(CH_3)-CO-$,

ainsi que leurs sels d'addition d'acide physiologiquement sans inconvénient.

2. La 6-(1-phényl-3-diméthylamino-propoxy)-flavone.

3. Procédé de préparation d'éthers basiques de formule générale I:

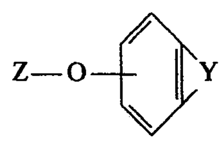

(I)

dans laquelle

Z représente $CH_3-NR^2-CH_2CH_2-CHR^1$, (1-$R^2$-3-pipéridyl)$-CHR^3-$, (1-$R^2$-2-pipéridyl)$-CH_2-CHR^3-$ ou 1-$R^2$-3-$R^4$-4-hexahydroazépinyle,
$R^1$ représente un groupe cyclopropyle ou Ar,
$R^2$ représente H, un groupe alkyle contenant 1 à 4 atomes de carbone, un groupe allyle, un groupe cycloalkylalkyle contenant 4 à 8 atomes de carbone ou un groupe benzyle,
$R^3$ représente H ou Ar,
$R^4$ représente H ou un groupe alkyle contenant 1 à 4 atomes de carbone,
Y représente $-O-CHQ^1-CHQ^2-CH_2-$, $-O-CHQ^1-CHQ^2-CO-$, $-O-CQ^1{=}CQ^2-CO-$ ou $-CH_2-CHQ^1-CHQ^2-CO-$,
$Q^1$ et $Q^2$ représentent chacun H, un groupe alkyle contenant 1 à 4 atomes de carbone, un groupe cycloalkyle contenant 3 à 6 atomes de carbone ou Ar, et
Ar représente un groupe phényle ou un groupe phényle substitué par F, Cl, un groupe alcoxy ou un groupe alkylthio contenant chacun 1 à 4 atomes de carbone, un groupe méthylène-dioxy ou par $CF_3$,

mais dans laquelle

$R^1$ représente un groupe cyclopropyle ou un groupe phényle substitué par F, Cl, un groupe alcoxy ou un groupe alkylthio contenant chacun 1 à 4 atomes de carbone, un groupe méthylène-dioxy ou par $CF_3$,

lorsque simultanément

Z représente un groupe $(CH_3)_2N-CH_2CH_2-CHR^1$ en position 7, et
Y représente $-O-C(C_6H_5){=}CH-CO$ ou $-O-C(C_6H_5){=}C(CH_3)-CO-$,

ainsi que de leurs sels d'addition d'acide physiologiquement sans inconvénient, caractérisé en ce qu'on fait réagir un phénol de formule générale II:

HO—G (II)

dans laquelle

G représente le groupe

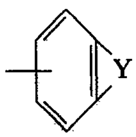

ou un de ses sels avec une amine de formulé générale III

Z—X (III)

dans laquelle

X représente Cl, Br, I ou OH, et
Z a la signification indiquée,

ou avec un de des dérivés réactifs et, éventuellement, dans un composé obtenu de formule I, par traitement avec un agent d'alkylation, d'allylation, de cycloalkylalkylation ou de benzylation, on transforme un groupe amino secondaire en un groupe amino tertiaire correspondant ou, par traitement avec un agent réducteur, on transforme un groupe N-benzyle en un groupe NH et/ou, par traitement avec un acide, on transforme une base obtenue de formule I en un de ses sels d'addition d'acide physiologiquement sans inconvénient.

4. Procédé en vue d'obtenir des préparations pharmaceutiques, caractérise en ce qu'on met, sous une forme de dosage appropriée, un composé de formule I et/ou un de ses sels d'addition d'acide physiologiquement sans inconvénient suivant la revendication 1 conjointement avec au moins une substance auxiliaire ou une substance support solide, liquide ou semi-liquide et éventuellement en combinaison avec une ou plusieurs autres substances actives.

5. Préparation pharmaceutique, caractérisée en ce qu'elle contient au moins un composé de formule générale I et/ou un de ses sels d'addition d'acide physiologiquement sans inconvénient suivant la revendication 1.